# EUROPEAN PATENT SPECIFICATION

(11) **EP 2 468 268 B1**
(45) Date of publication and mention of the grant of the patent: **13.12.2017**
(21) Application number: 11194832.9
(22) Date of filing: 21.12.2011
(51) Int. Cl.: A61K 9/20, A61K 9/28, A61K 31/395

(54) **Combination composition of vildagliptin and gliclazide**
Kombinationszusammensetzung aus Vildagliptin und Gliclazid
Composition de combinaison de vildagliptine et de gliclazide

(30) Priority: 21.12.2010 TR 201010683; 29.07.2011 TR 201107482; 01.08.2011 TR 201107563
(43) Date of publication of application: 27.06.2012
(73) Proprietor: Sanovel Ilaç Sanayi Ve Ticaret Anonim Sirketi, 34460 Sariyer/Istanbul (TR)
(72) Inventor: Cifter, Ümit, 34398 Istanbul (TR); Türkyilmaz, Ali, 34398 Istanbul (TR); Yelken, Gülay, 34398 Istanbul (TR); Saydam, Mehtap, 34398 Istanbul (TR)
(74) Representative: Sevinç, Erkan

(56) References cited:
- WO-A1-2008/028914
- WO-A2-2010/092163
- US-A1- 2007 172 525
- GARBER A J ET AL: "Effects of vildagliptin on glucose control in patients with type 2 diabetes inadequately controlled with a sulphonylurea", DIABETES, OBESITY AND METABOLISM, BLACKWELL SCIENCE, vol. 10, no. 11, 1 November 2008 (2008-11-01), pages 1047-1056, XP009149673, ISSN: 1462-8902, DOI: 10.1111/J.1463-1326.2008.00859.X
- DATABASE WPI Week 201127 Thomson Scientific, London, GB; AN 2011-A18673 XP002670717, & CN 101 897 696 A (SHENZHEN AUSA PHARMED CO LTD) 1 December 2010 (2010-12-01)

## Description

### Field of Invention

The present invention relates to formulations made with vildagliptin or a pharmaceutically acceptable salt of vildagliptin and gliclazide or a pharmaceutically acceptable salt of gliclazide. The present invention particularly relates to a combination composition made of pellets and granules comprising vildagliptin and gliclazide.

### Background of Invention

Vildagliptin is a dipeptidyl dipeptidase-IV (DPP-IV) inhibitor developed for use in the treatment of type 2 diabetes (non-insulin dependent diabetes). Vildagliptin inhibits the degradation of the dipeptidyl dipeptidase-IV enzyme, thereby inhibiting the effects of incretin hormones, glucagon-like peptide-1 (GLP-1), and of glucose-dependent insulinotropic peptide (GIP). The chemical designation of vildagliptin is (S)-{[(3-hydroxyadamantan-1-yl)amino]acetyl}pyrrolidine-2-carbonitrile, with the chemical structure illustrated below in Formula 1.

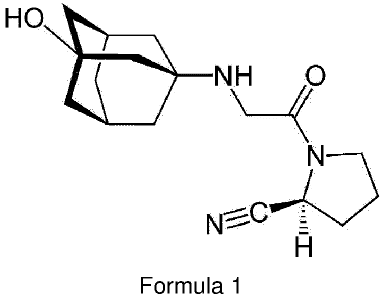

Vildagliptin is soluble in water and in organic polar solvents.

Vildagliptin is marketed under the trademark Galvus® in 50 mg dosage forms. It is used against diabetes mellitus, but particularly in treating type 2 diabetes.

There are various patents in the patent literature in relation to vildagliptin.

The patent application WO0034241 discloses vildagliptin or an acid addition salt thereof, as well as their use in diabetes mellitus and obesity.

The patent application WO2006078593 claims a direct-compression formulation of a DPP-IV inhibitor compound, and preferably of vildagliptin or an acid addition salt thereof.

The patent application WO2006135723 discloses a formulation, comprising vildagliptin as an active agent, as well as hydroxypropyl methyl cellulose, microcrystalline cellulose, and magnesium stearate.

Stability-related problems do occur in many active agents, including vildagliptin, under the influence of ambient and physical conditions. Vildagliptin is an active agent that is highly-susceptible to air and humidity. When vildagliptin is exposed to air and humidity, it degrades structurally and develops chemical behavioral changes. The stability of vildagliptin products developed is not at a desired level and the shelf life thereof is shortened. In addition, vildagliptin is reactive against the excipients employed in developing formulations containing the same. This fact causes impurities to occur in the formulation and leads to the inclusion of undesired components into the formulation.

Sulfonylurea is a name defining a group of orally administered anti-diabetic medicaments used in the treatment of type II diabetes mellitus. Diabetes mellitus is a disorder of the carbohydrate metabolism, in which insulin secretion is diminished, or which occurs due to a varying insulation secretion or decreased insulin activity or a combination of both factors. It is believed that sulfonylureas stimulate the insulin secretion from pancreatic islet cells in the mediation of receptors reported to be adenosine 5'-triphosphate-sensitive potassium channels. For this reason, sulfonylureas basically increase the endogenous insulin secretion so that an efficient control is provided in the blood sugar levels, not controllable via diets, of the diabetics and particularly of the type II diabetics.

Sulfonylureas are considered to be divided into two categories: first generation agents, e.g. tolbutamide, chlorpropamide, tolazamide, acetohexamide; and second generation agents, e.g. glyburide (glibenclamide), glipizide, gliclazide.

Gliclazide N-(4-methylbenzenesulfonyl)-N'-(3-azabicyclo[3.3.0]-oct-3-yl)urea), one type of second generation sulfonylureas, is an active agent which has antidiabetic properties at typical doses administered to humans (its chemical structure is illustrated with Formula 1).

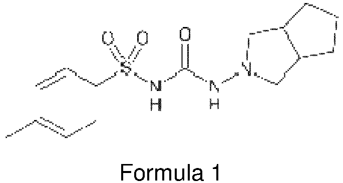

Gliclazide has been administered in the form of 80 mg conventional tablets until today. It is usually administrated one tablet twice a day, making a total of two tablets. It may be altered, however, to 1 to 4 tablets a day, as required by the severity of the diabetes case. A 30 mg modified-release dosage form is also available at the markets.

The formulation of gliclazide was first disclosed on 10.12.1996 with the patent document US 3,501,495 (SCIENCE UNION ET CIE. SOC.), disclosing the formula N-(4-methylbenzenesulphonyl)-N'-(3-azabicyclo[3.3.0]-oct-3-yl)urea), which has hypoglycemic properties, and is orally used in the treatment of diabetes mellitus.

Immediate-release formulations of sulfonylurea have been disclosed in many different patents in the past.

The patent US 4,696,815 discloses immediate-release tablets comprising sulfonylurea, formulated with an acidified and/or alkalized excipient and an inert polar solvent like polyethylene glycol. An analogous immediate-release formulation comprising an acidified and/or alkalized excipient, an inert polar solvent, and polyvinylpyrrolidone is also described in that patent.

The patent US 6,733,782 discloses a core tablet for the controlled-release of gliclazide, ensuring a sustained and constant release of the active agent by making use of hydroxypropylmethyl cellulose (HPMC) which is not influenced from the pH variations of the solubilizing medium following an oral administration. The main target of that invention is to obtain an oral dosage form, which can be administered once a day and provides extended release.

In the patent US 6,703,045 is disclosed an oral controlled-release system, which is beneficial in lowering serum glucose levels. That patent also describes a method of lowering serum glucose levels with an oral controlled-release dosage form comprising glipizide, and that once-a-day dosage form ensures to keep the medicament at a therapeutic level throughout the day.

The patent EP 1 741 435 A1 relates to an oral modified-release tablet composition comprising a pharmaceutically effective amount of a micronized active ingredient for lowering blood glucose level and a hydrophilic polymer, as well as to a method of preparing a novel composition for that modified-release oral tablet.

With the application WO 2006/061697 A1 is disclosed sustained-release pharmaceutical formulations suitable for once-a-day administration, as well as a process for preparing such formulations comprising sulfonylurea, polymer, disaccharide and/or monosaccharide (lactose, sucrose, maltose, galactose, trehalose, maltitol, dextrose or the mixtures thereof) exhibiting a drug release profile substantially independent of pH of the dissolution medium in pH range 4 to 8.

The gliclazide active agent is quite susceptible to humidity. Particularly if it is formulated with polymers having a hydrophilic structure, extreme stability problems are encountered. While it is desired to obtain a desired release profile with hydrophilic polymers, the affinity of this polymer to humidity brings about stability-related problems. This is unfavorable in terms of pharmaceutics. Therefore, a formulation is needed that would overcome this problem.

One of the problems encountered in formulations relates to the dissolution rate and solubility. Increasing the dissolution rate and solubility is directly associated with bioequivalence. Therefore, these parameters need to be improved.

Making an assessment within the scope of said problems, it becomes obvious that a need exists for a stable combination composition of vildagliptin and gliclazide, which would provide a proper release profile.

### Object and Brief Description of Invention

The present invention provides a combination composition, eliminating all aforesaid problems and bringing additional advantages to the relevant prior art.

Accordingly, the main object of the present invention is to obtain a formulation, which is orally administered once or twice a day.

Another object of the present invention is to reduce the treatment onset time, with said formulation providing a 24-hour effect.

A further object of the present invention is to obtain a stable combination composition.

Another object of the present invention is to obtain a combination composition which provides the desired release profile.

A further object of the present invention is to obtain a combination composition having the desired levels of dissolution rate and solubility respectively.

A combination composition is developed to achieve all objects, referred to above and to emerge from the following detailed description.

In a preferred embodiment according to the present invention, said novelty is characterized in that said composition comprises particles containing vildagliptin or a pharmaceutically acceptable salt or polymorph of vildagliptin, and particles containing gliclazide or a pharmaceutically acceptable salt or polymorph of gliclazide.

A preferred embodiment of the present invention also contains one or more excipient(s).

In a preferred embodiment of the present invention, said particle is selected from pellet, granule, or powder forms.

In an embodiment of the present invention, at least a portion of said pellets or granules provides immediate release, whereas the other portion provides controlled release.

In a preferred embodiment of the present invention, the entirety of said pellets or granules provide immediate release.

In a preferred embodiment of the present invention, the entirety of said pellets or granules provide controlled release.

In a preferred embodiment of the present invention, said pellets are sugar pellets.

In a preferred embodiment of the present invention, said pellets are formed by a sphere formation process of at least a portion of said composition. The components gliclazide and dibasic calcium phosphate of the composition are sieved and mixed in a high-shear granulator. This is granulated with an ethyl cellulose/polyvinylpyrrolidone solution. The formed granules are extruded into spheres to give pellets.

In a preferred embodiment according to the present invention, said excipient(s) comprise(s) at least one or a mixture of controlled-release providing agents, fillers, binders, disintegrant, glidants, lubricants.

In a preferred embodiment of the present invention, said controlled-release providing agent comprises at least one or a properly-proportioned mixture of ethyl cellulose, polymethacrylate, polyethylene oxide, glyceryl behenate, hydroxypropyl methyl cellulose, hydroxypropyl cellulose, xanthan gum, stearoyl polyoxyl glycerides, and sodium alginate.

In a preferred embodiment of the present invention, said filler comprises at least one or a mixture of starch, mannitol, dibasic calcium phosphate, lactose, starch, calcium hydrogen phosphate dihydrate, dicalcium hydrogen phosphate anhydrate, calcium phosphate trihydrate, and glucose; but is preferably selected from mannitol and/or dibasic calcium phosphate.

In a preferred embodiment according to the present invention, the particle size of said pellets or granules are the range of 400 to 2000 µm, preferably 400 to 1500 µm, more preferably 450 to 1000 µm.

In a preferred embodiment according to the present invention, the particle size of said pellets or granules are the range of 500 to 700 µm.

In a preferred embodiment of the present invention, said binder is polyvinylpyrrolidone.

In a preferred embodiment of the present invention, said disintegrant is croscarmellose sodium.

In a preferred embodiment of the present invention, said glidant is at least one or a mixture of colloidal silicon dioxide, talk, aluminum silicate, magnesium silicate, with colloidal silicon dioxide being preferred.

In a preferred embodiment of the present invention, said lubricant is magnesium stearate.

A preferred embodiment of the present invention consist of
a. immediate-release layer
   i. vildagliptin at 3.5 to 60% by weight,
   ii. mannitol at 5 to 90% by weight,
   iii. croscarmellose sodium at 6 to 25% by weight,
   iv. polyvinylpyrrolidone at 0.1 to 25% by weight,
   v. silicon dioxide at 0.1 to 0.2% by weight,
   vi. magnesium stearate at 0.25 to 2% by weight;
b. controlled-release layer
   i. gliclazide at 15 to 60% by weight,
   ii. polyvinylpyrrolidone at 0.1 to 25% by weight,
   iii. dibasic calcium phosphate at 5-90% by weight,
   iv. ethyl cellulose or polymethacrylate at 0.5 to 40% by weight,
   v. triethyl citrate at 1 to 40% by weight,
   vi. silicon dioxide at 0.1 to 0.2% by weight,
   vii. magnesium stearate at 0.25 to 2% by weight;
c. coating layer
   i. hydroxypropyl methyl cellulose capsule at 1 to 5% by weight.

Another preferred embodiment of the present invention consist of
a. controlled-release layer
   i. vildagliptin at 3.5 to 60% by weight,
   ii. mannitol at 5 to 90% by weight,
   iii. polyvinylpyrrolidone at 0.1 to 25% by weight,
   iv. silicon dioxide at 0.1 to 0.2% by weight,
   v. magnesium stearate at 0.25 to 2% by weight;
b. controlled-release layer
   i. gliclazide at 15 to 60% by weight,
   ii. polyvinylpyrrolidone at 0.1 to 25% by weight,
   iii. sugar pellet at 5 to 90% by weight,
   iv. polymethacrylate or derivatives thereof at 0.5 to 40% by weight (pH independent),
   v. diethyl phthalate at 1 to 40% by weight,
   vi. silicon dioxide at 0.1 to 0.2% by weight,
   vii. magnesium stearate at 0.25 to 2% by weight;
c. coating layer
   i. hydroxypropyl methyl cellulose capsule at 1 to 5% by weight.

A further preferred embodiment of the present invention provides a method for preparing a pharmaceutical formulation, this method comprising the steps of
a. sieving vildagliptin, croscarmellose sodium, and mannitol, and mixing in a high-shear granulator,
b. granulating the resulting mixture with an alcoholic/hydroalcoholic polyvinylpyrrolidone solution,
c. sieving the wet granules formed, drying the same, and sieving the dried granules again,
d. sieving gliclazide and dibasic calcium phosphate, and mixing in a high-shear granulator,
e. granulating the resulting mixture with a hydroalcoholic ethyl cellulose/polyvinylpyrrolidone solution,
f. extruding the formed granules into spheres to give pellets,
g. coating the pellets obtained above with a solution of alcoholic/hydroalcoholic ethyl cellulose/polymethacrylate containing triethyl citrate,
h. adding first silicon dioxide and then magnesium stearate to the vildagliptin-containing granules and gliclazide-containing coated pellets and mixing the resulting mixture,
i. filling the granules and pellets obtained above into capsules.

A further preferred embodiment of the present invention provides a method for preparing a pharmaceutical formulation, this method comprising the steps of
a. sieving vildagliptin, croscarmellose sodium, and mannitol, and mixing in a high-shear granulator,
b. granulating the resulting mixture with an alcoholic/hydroalcoholic polyvinylpyrrolidone solution,
c. sieving the wet granules formed, drying the same, and sieving the dried granules again,
d. preparing a hydroalcoholic polyvinylpyrrolidone solution of gliclazide and coating the sugar pellets with this solution,
e. preparing an alcoholic/hydroalcoholic solution of ethyl cellulose/polymethacrylate and diethyl phthalate, and coating the gliclazide-containing sugar pellets,
f. adding first silicon dioxide and then magnesium stearate to the vildagliptin-containing granules and gliclazide-containing coated pellets and mixing the resulting mixture,
g. filling the granules and pellets obtained above into capsules.

### Detailed Description of Invention

### Example 1

| **Immediate-release phase** | **weight %** |
|---|---|
| vildagliptin | 3.5 - 60 |
| mannitol | 5.0 - 90 |
| croscarmellose sodium | 6 - 25 |
| polyvinylpyrrolidone | 0.1 - 25 |
| silicon dioxide | 0.1 - 0.2 |
| magnesium stearate | 0.25 - 2.0 |

| **Controlled-release phase** | |
|---|---|
| gliclazide | 15 - 60 |
| polyvinylpyrrolidone | 0.1 - 25 |
| sugar pellet | 5 - 90 |
| polymethacrylate or derivatives thereof | 0.5 - 40 |
| diethyl phthalate | 1 - 40 |
| silicon dioxide | 0.1 - 0.2 |
| magnesium stearate | 0.25 - 2.0 |

| **Coating layer** | |
|---|---|
| hydroxypropyl methyl cellulose capsule | 1 - 5 |

### Production Method:

Vildagliptin, croscarmellose sodium, and mannitol are sieved and mixed in a high-shear granulator. The resulting mixture is granulated with an alcoholic/hydroalcoholic polyvinylpyrrolidone solution. Wet granules formed are sieved, then dried and the dried granules are sieved again. Then, gliclazide and dibasic calcium phosphate are sieved and mixed in a high-shear granulator. The resulting mixture is granulated with an hydroalcoholic ethyl cellulose/polyvinylpyrrolidone solution. The formed granules are extruded into spheres to give pellets. The pellets obtained are coated with a solution of alcoholic/hydroalcoholic ethyl cellulose/polymethacrylate containing triethyl citrate. First silicon dioxide and then magnesium stearate are added to the vildagliptin-containing granules and gliclazide-containing coated pellets and the resulting mixture is mixed. The granules and pellets obtained are filled into capsules to complete the process.

### Example 2

| **Controlled-release phase 1** | **weight %** |
|---|---|
| vildagliptin | 3.5 - 60 |
| mannitol | 5.0 - 90 |
| polyvinylpyrrolidone | 1 - 25 |
| silicon dioxide | 0.1 - 0.2 |
| magnesium stearate | 0.25 - 2.0 |

| **Controlled-release phase 2** | |
|---|---|
| gliclazide | 15 - 60 |
| sugar pellet | 5.0 - 90 |
| polymethacrylate or derivatives thereof | 0.5 - 40 |
| polyvinylpyrrolidone | 1 - 25 |
| silicon dioxide | 0.1 - 0.2 |
| magnesium stearate | 0.25 - 2.0 |
| hydroxypropyl methyl cellulose capsule | 1 - 5 |

### Production Method:

Vildagliptin, croscarmellose sodium, and mannitol are sieved, and then mixed in a high-shear granulator. This mixture is granulated with an alcoholic/hydroalcoholic polyvinylpyrrolidone solution. Wet granules formed are sieved, then dried and the dried granules are sieved again. A hydroalcoholic polyvinylpyrrolidone solution of gliclazide is prepared and the sugar pellets are coated with this solution. An alcoholic/hydroalcoholic solution of ethyl cellulose/polymethacrylate and diethyl phthalate is prepared, and the gliclazide-containing sugar pellets are coated with this solution. First silicon dioxide and then magnesium stearate are added to the vildagliptin-containing granules and gliclazide-containing coated pellets and the resulting mixture is mixed. The granules and pellets obtained are filled into capsules to complete the process.

With this invention realized, a combination composition is obtained which has a surprising stability and release profile. Said composition is characterized by comprising particles containing vildagliptin or a pharmaceutically acceptable salt or polymorph of vildagliptin, and particles containing gliclazide or a pharmaceutically acceptable salt or polymorph of gliclazide. Particularly the pellets comprised in this composition augment the composition's dissolution rate and solubility to a desired level. Keeping the particle size of the pellets or granules between 400-2000 µm, preferably 400 to 1500 µm, more preferably 450 to 1000 µm bears significance in realizing the desired effect.

The formulation developed is used in the treatment of diabetes mellitus.

The combination composition includes pellet formulations, as well as separate use, fixed dose and kit formulations.

It is further possible to use the following additional excipients in this formulation.

Fillers, but are not restricted to at least one or a mixture of starch, mannitol, sorbitol, sucrose, inorganic salts, calcium salts, polysaccharides, dextrose, dicalsium phosphate, sodium chloride, dextrates, lactitol, maltodextrin, sucrose-maltodextrin mixtures, xylitol, trehalose, etc..

Binders, but are not restricted to at least one or a mixture of polyvinylpyrrolidone, gelatin, sugars, glucose, natural gums, gums, synthetic celluloses, polymethacrylate, hydroxypropylmethyl cellulose, hydroxypropyl cellulose, carboxymethyl cellulose, methyl cellulose, and other cellulose derivatives, etc..

Glidants, but are not restricted to at least one or a mixture of colloidal silicon dioxide, talk, and aluminum silicate.

Lubricants, but are not restricted to at least one or a mixture of sodium stearyl fumarate, magnesium stearate, polyethylene glycol, stearic acid, metal stearates, boric acid, sodium chloride benzoate and acetate, sodium or magnesium lauryl sulfate, etc..

Disintegrants, but are not restricted to at least one or a mixture of sodium starch glycolate, croscarmellose sodium, crospovidone, sodium alginate, gums, starch, and magnesium aluminum silicate, etc..

Hot melts, but are not restricted to Poloxamer 188 (polyoxyethylene-polyoxypropylene block copolymer), Gelucire 50/13 (stearyl macrogolglyceride), polyethylene glycol, povidone, Soluplus, cationic methacrylate, copovidone, methacrylic acid copolymers, cellulose acetate phthalate, acetyl monoglyceride, dibutyl tartrate, diethyl phthalate, dimethyl phthalate, glycerin, propylene glycol, triacetine, triacetine citrate and Tripropionin, etc..

Surface active agents, but are not restricted to at least one or a mixture of sodium lauryl sulfate, dioctyl sulfosuccinate, polysorbates and polyoxyethylene alkyl esters and ethers thereof, glyceryl monolaurate saponins, sorbitan laurate, sodium lauryl sulfate, and magnesium lauryl sulfate, etc..

Coating agents, but are not restricted to e.g. hydroxypropyl methyl cellulose, polyethylene glycol, polyvinylpyrrolidone, polyvinylpyrrolidone-vinyl acetate copolymer (PVP-VA), polyvinyl alcohol and other polymers, and all kinds of OpadryTM, as well as pigments, dyes, titanium dioxide and iron oxide, talk, etc..

The protection scope of the present invention is set forth in the annexed claims and cannot be restricted to the illustrative disclosures given above, under the detailed description. Any alternative embodiments to be produced by those skilled in the art according to the basic principles, which are under the protection scope as set forth in the claims, shall be an infringement of the present invention.

## Claims

1. A combination composition, **characterized by** comprising particles containing vildagliptin or a pharmaceutically acceptable salt or polymorph of vildagliptin, and particles containing gliclazide or a pharmaceutically acceptable salt or polymorph of gliclazide wherein particles or granules in gliclazide part are in a controlled release form.

2. The combination composition according to Claim 1, further comprising at least one or more excipient.

3. The combination composition according to any of the preceding claims, wherein said particle is selected from the forms of pellets, granules, and powders.

4. The combination composition according to any of the preceding claims, wherein entirety of the particles is in a controlled release form.

5. The combination composition according to any of the preceding claims, wherein said excipients comprise at least one or a mixture of controlled-release providing agents, fillers, binders, disintegrants, glidants, and lubricants.

6. The combination composition according to any of the preceding claims, wherein said pellets are sugar pellets.

7. The combination composition according to any of the preceding claims, wherein said pellets are formed by a sphere formation process of at least a portion of said composition.

8. The combination composition according to any of the preceding claims, wherein said controlled-release providing agent is at least one or a mixture of ethyl cellulose, polymethacrylate, polyethylene oxide, glyceryl behenate, hydroxypropyl methyl cellulose, hydroxypropyl cellulose, xanthan gum, stearoyl polyoxyl glycerides, and sodium alginate.

9. The combination composition according to any of the preceding claims, wherein said filler is at least one or a mixture of starch, mannitol, dibasic calcium phosphate, lactose, starch, calcium hydrogen phosphate dihydrate, dicalcium hydrogen phosphate anhydrate, calcium phosphate trihydrate, silicon dioxide, and glucose; with mannitol and/or dibasic calcium phosphate being preferred.

10. The combination composition according to any of the preceding claims, wherein the particle size of said pellets is in the range of 400 to 2000 µm, preferably 400 to 1500 µm, more preferably 450 to 1000 µm.

11. The combination composition according to any of the preceding claims, wherein said binder is polyvinylpyrrolidone.

12. The combination composition according to any of the preceding claims, wherein said disintegrant is croscarmellose sodium.

13. The combination composition according to any of the preceding claims, wherein said glidant is at least one or a mixture of colloidal silicon dioxide, talk, aluminum silicate, and magnesium silicate; with colloidal silicon dioxide being preferred.

14. The combination composition according to any of the preceding claims, wherein said lubricant is magnesium stearate.

15. The combination composition according to any of the preceding claims, consisting of
a. immediate-release layer
i. vildagliptin at 3.5 to 60% by weight,
ii. mannitol at 5 to 90% by weight,
iii. croscarmellose sodium at 6 to 25% by weight,
iv. polyvinylpyrrolidone at 0.1 to 25% by weight,
v. silicon dioxide at 0.1 to 0.2% by weight,
vi. magnesium stearate at 0.25 to 2% by weight;
b. controlled-release layer
i. gliclazide at 15 to 60% by weight,
ii. polyvinylpyrrolidone at 0.1 to 25% by weight,
iii. dibasic calcium phosphate at 5 to 90% by weight,
iv. ethyl cellulose or polymethacrylate at 0.5 to 40% by weight,
v. triethyl citrate at 1 to 40% by weight,
vi. silicon dioxide at 0.1 to 0.2% by weight,
vii. magnesium stearate at 0.25 to 2% by weight;
c. coating layer
i. hydroxypropyl methyl cellulose capsule at 1 to 5% by weight.

16. The combination composition according to any of the preceding claims, consisting of
a. controlled-release layer
i. vildagliptin at 3.5 to 60% by weight,
ii. mannitol at 5 to 90% by weight,
iii. polyvinylpyrrolidone at 0.1 to 25% by weight,
iv. silicon dioxide at 0.1 to 0.2% by weight,
v. magnesium stearate at 0.25 to 2% by weight;
b. controlled-release layer
i. gliclazide at 15 to 60% by weight,
ii. polyvinylpyrrolidone at 0.1 to 25% by weight,
iii. sugar pellet at 5 to 90% by weight,
iv. polymethacrylate or derivatives thereof at 0.5 to 40% by weight (pH independent),
v. diethyl phthalate at 1 to 40% by weight,
vi. silicon dioxide at 0.1 to 0.2% by weight,
vii. magnesium stearate at 0.25 to 2% by weight;
c. coating layer
i. hydroxypropyl methyl cellulose capsule at 1 to 5% by weight.

17. A method for preparing a pharmaceutical formulation according to any of the preceding claims, comprising the steps of
a. sieving vildagliptin, croscarmellose sodium, and mannitol, and mixing in a high-shear granulator,
b. granulating the resulting mixture with an alcoholic/hydroalcoholic polyvinylpyrrolidone solution,
c. sieving the wet granules formed, drying the same, and sieving the dried granules again,
d. sieving gliclazide and dibasic calcium phosphate, and mixing in a high-shear granulator,
e. granulating the resulting mixture with a hydroalcoholic ethyl cellulose/polyvinylpyrrolidone solution,
f. extruding the formed granules into spheres to give pellets,
g. coating the pellets obtained above with a solution of alcoholic/hydroalcoholic ethyl cellulose/polymethacrylate containing triethyl citrate,
h. adding first silicon dioxide and then magnesium stearate to the vildagliptin-containing granules and gliclazide-containing coated pellets and mixing the resulting mixture,
i. filling the granules and pellets obtained above into capsules.

18. A method for preparing a pharmaceutical formulation according to any of the preceding claims, comprising the steps of
a. sieving vildagliptin, croscarmellose sodium, and mannitol, and mixing in a high-shear granulator,
b. granulating the resulting mixture with an alcoholic/hydroalcoholic polyvinylpyrrolidone solution,
c. sieving the wet granules formed, drying the same, and sieving the dried granules again,
d. preparing a hydroalcoholic polyvinylpyrrolidone solution of gliclazide and coating the sugar pellets with this solution,
e. preparing an alcoholic/hydroalcoholic solution of ethyl cellulose/polymethacrylate and diethyl phthalate, and coating the gliclazide-containing sugar pellets,
f. adding first silicon dioxide and then magnesium stearate to the vildagliptin-containing granules and gliclazide-containing coated pellets and mixing the resulting mixture,
g. filling the granules and pellets obtained above into capsules.

19. The combination composition according to any of the preceding claims for preventing or treating diabetes mellitus in mammalians and particularly in humans.

## Patentansprüche

1. Kombinationszusammensetzung, **dadurch gekennzeichnet, dass** sie Partikel umfasst, die Vildagliptin oder ein pharmazeutisch verträgliches Salz oder ein Polymorph von Vildagliptin enthalten und Partikel, die Gliclazid oder ein pharmazeutisch verträgliches Salz oder ein Polymorph von Gliclazid enthalten, wobei die Partikel oder Granulate im Gliclazid-Teil in einer Form mit kontrollierter Freisetzung vorliegen.

2. Kombinationszusammensetzung nach Anspruch 1, ferner umfassend mindestens einen oder mehrere Exzipienten.

3. Kombinationszusammensetzung nach einem der vorhergehenden Ansprüche, wobei der Partikel ausgewählt ist aus den Formen von Pellets, Granulaten und Pulvern.

4. Kombinationszusammensetzung nach einem der vorhergehenden Ansprüche, wobei die Gesamtheit der Partikel in einer Form mit kontrollierter Freisetzung vorliegt.

5. Kombinationszusammensetzung nach einem der vorhergehenden Ansprüche, wobei die Exzipienten mindestens eines oder eine Mischung der Mitteln aufweisen, die Bereitstellungsmittel für eine kontrollierte Freisetzung, Füllstoffe, Bindemittel, Desintegrationsmittel, Fließmittel und Schmiermittel umfassen.

6. Kombinationszusammensetzung nach einem der vorhergehenden Ansprüche, wobei die Pellets Zuckerpellets sind.

7. Kombinationszusammensetzung nach einem der vorhergehenden Ansprüche, wobei die Pellets aus mindestens einem Teil der Zusammensetzung durch einen sphärischen Formationsprozess gebildet werden.

8. Kombinationszusammensetzung nach einem der vorhergehenden Ansprüche, wobei das Bereitstellungsmittel für die kontrollierte Freisetzung aus mindestens einem oder einer Mischung der Stoffe Ethylcellulose, Polymethacrylat, Polyethylenoxid, Glycerylbehenat, Hydroxypropyl methyl cellulose, Hydroxypropyl cellulose, Xanthan, Stearoyl Polyoxylglycerid und Natriumalginat besteht.

9. Kombinationszusammensetzung nach einem der vorhergehenden Ansprüche, wobei der Füllstoff aus mindestens einem der Stoffe Stärke, Mannit, zweibasigem calcium phosphat, Laktose, Stärke, Calciumhydrogenphosphat Dihydrat, Dicalciumhydrogenphosphat Anhydrat, Calciumphosphate Trihydrat, Siliziumdioxid und Glucose oder einer Mischung aus diesen besteht; wobei Mannit und/oder zweibasiges Calciumphosphat bevorzugt ist.

10. Kombinationszusammensetzung nach einem der vorhergehenden Ansprüche, wobei die Partikelgröße der Pellets im Bereich von 400 bis 2000 µm, bevorzugt im Bereich von 400 bis 1500 µm, besonders bevorzugt im Bereich von 450 bis 1000 µm liegt.

11. Kombinationszusammensetzung nach einem der vorhergehenden Ansprüche, wobei das Bindemittel Polyvinylpyrrolidon ist.

12. Kombinationszusammensetzung nach einem der vorhergehenden Ansprüche, wobei das Desintegrationsmittel Croscarmellose-Natrium ist.

13. Kombinationszusammensetzung nach einem der vorhergehenden Ansprüche, wobei das Fließmittel aus mindestens einem der Stoffe kolloidales Siliziumdioxid, Talk, Aluminiumsilikat und Magnesiumsilikat oder einer Mischung aus diesen besteht; wobei kolloidales Siliziumdioxid bevorzugt ist.

14. Kombinationszusammensetzung nach einem der vorhergehenden Ansprüche, wobei das Formtrennmittel Magnesium-Stearat ist.

15. Kombinationszusammensetzung nach einem der vorhergehenden Ansprüche, bestehend aus einer
a. Sofortfreigabe Schicht
i. Vildagliptin von 3,5 bis 60 Gew.-%,
ii. Mannit von 5 bis 90 Gew.-%
iii. Croscarmellose-Natrium von 6 bis 25 Gew.-%
iv. Polyvinylpyrrolidon von 0,1 bis 25 Gew.-%
v. Siliziumdioxid von 0,1 bis 0,2 Gew.-%
vi. Magnesium-Stearat von 0,25 bis 2 Gew.-%
b. Kontrollierten Freisetzungs-Schicht
i. Gliclazid von 15 bis 60 Gew.-%
ii. Polyvinylpyrrolidon von 0,1 bis 25 Gew.-%
iii. Zweibasigem Calciumphosphat von 5 bis 90 Gew.-%
iv. Ethylcellulose oder Polymethacrylat von 0,5 bis 40 Gew.-%
v. Triethylcitrat von 1 bis 40 Gew.-%
vi. Siliziumdioxid von 0,1 bis 0,2 Gew.-%
vii. Magnesium-Stearat von 0,25 bis 2 Gew.-%
c. Beschichtung
i. Hydroxypropyl methyl cellulose Kapseln von 1 bis 5 Gew.-%

16. Kombinationszusammensetzung nach einem der vorhergehenden Ansprüche, bestehend aus einer
a. Kontrollierten Freisetzungs-Schicht
i. Vildagliptin von 3,5 bis 60 Gew.-%
ii. Mannitol von 5 bis 90 Gew.-%
iii. Polyvinylpyrrolidon von 0,1 bis 25 Gew.-%
iv. Siliziumdioxid von 0,1 bis 0,2 Gew.-%
v. Magnesium-Stearat von 0,25 bis 2 Gew.-%
b. Kontrollierten Freisetzungs-Schicht
i. Gliclazid von 15 bis 60 Gew.-%
ii. Polyvinylpyrrolidon von 0,1 bis 25 Gew.-%
iii. Zuckerpellets von 5 bis 90 Gew.-%
iv. Polymethacrylat oder Derivate davon von 0,5 bis 40 Gew.-% (pH unabhängig)
v. Diethylphtalat von 1 bis 40 Gew.-%
vi. Siliziumdioxid von 0,1 bis 0,2 Gew.-%
vii. Magnesium-Stearat von 0,25 bis 2 Gew.-%
c. Beschichtung
i. Hydroxypropyl methyl cellulose Kapseln von 1 bis 5 Gew.-%

17. Verfahren für die Bereitstellung einer pharmazeutischen Formulierung nach einem der vorhergehenden Ansprüche, umfassend die Schritte
a. Sieben von Vildagliptin, Croscarmellose-Natrium und Mannit und Mischen in einem Granulator mit großer Scherung,
b. Granulierung der resultierenden Mischung mit einer alkoholischen/hydroalkoholischen Polyvinylpyrrolidon Lösung,
c. Sieben der geformten nassen Granulaten, Trocknung derselben und wiederholtes Sieben der getrockneten Granulate,
d. Sieben von Gliclazid und zweibasigem Calciumphosphat und Mischen in einem Granulator mit großer Scherung,
e. Granulierung der resultierenden Mischung mit einer hydroalkoholischen Ethylcellulose/Polyvinylpyrrolidon Lösung,
f. Extrudieren der geformten Granulate in sphärische Pellets,
g. Beschichtung der oben erhaltenen Pellets mit einer alkoholischen/hydroalkolischen Ethylcellulose/Polymethacrylat Lösung enthaltend Triethylcitrat,
h. Zuerst eine Zugabe von Siliziumdioxid und dann Magnesium-Stearat zu den Vildagliptin enthaltenen Granulaten und Gliclazid enthaltenden beschichteten Pellets und Mischen der resultierenden Mischung,
i. Füllen der oben erhaltenen Granulate und Pellets in Kapseln.

18. Verfahren für die Bereitstellung einer pharmazeutischen Formulierung nach einem der vorhergehenden Ansprüche, umfassend die Schritte
a. Sieben von Vildagliptin, Croscarmellose-Natrium und Mannit und Mischen in einem Granulator mit großer Scherung,
b. Granulierung der resultierenden Mischung mit einer alkoholischen/hydroalkoholischen Polyvinylpyrrolidon Lösung,
c. Sieben der geformten nassen Granulate, Trocknung derselben und wiederholtes Sieben der getrockneten Granulate,
d. Vorbereiten einer hydroalkoholischen Polyvinylpyrrolidon Lösung von Gliclazid und Beschichtung der Zuckerpellets mit dieser Lösung,
e. Vorbereiten einer alkoholischen/hydroalkoholischen Lösung von Ethylcellulose/Polymethacrylat und Diethylphtalat und Beschichtung der Gliclazid enthaltenen Zuckerpellets,
f. Zuerst eine Zugabe von Siliziumdioxid und dann Magnesium Stearat zu der Vildagliptin enthaltenen Granulaten und Gliclazid enthaltenden beschichteten Pellets und Mischen der resultierenden Mischung,
g. Füllen der oben erhaltenen Granulate und Pellets in Kapseln.

19. Kombinationszusammensetzung nach einem der vorhergehenden Ansprüche zur Vorbeugung oder Behandlung von Diabetes mellitus bei Säugetieren und besonders bei Menschen.

## Revendications

1. Composition de combinaison, **caractérisée par le fait qu'**elle comprend des particules contenant de la vildagliptine ou un sel ou polymorphe pharmaceutiquement acceptable de vildagliptine, et des particules contenant du gliclazide ou un sel ou polymorphe pharmaceutiquement acceptable de gliclazide, les particules ou granules dans la partie gliclazide se trouvant dans une forme à libération contrôlée.

2. Composition de combinaison selon la revendication 1, comprenant en outre au moins un ou plusieurs excipients.

3. Composition de combinaison selon l'une quelconque des revendications précédentes, dans laquelle lesdites particules sont choisies parmi les formes de pastilles, de granules et de poudres.

4. Composition de combinaison selon l'une quelconque des revendications précédentes, dans laquelle la totalité des particules se trouve dans une forme à libération contrôlée.

5. Composition de combinaison selon l'une quelconque des revendications précédentes, dans laquelle lesdits excipients comprennent au moins un ou un mélange d'agents assurant une libération contrôlée, de charges, de liants, de désintégrants, d'agents de glissement et de lubrifiants.

6. Composition de combinaison selon l'une quelconque des revendications précédentes, dans laquelle lesdites pastilles sont des pastilles de sucre.

7. Composition de combinaison selon l'une quelconque des revendications précédentes, dans laquelle lesdites pastilles sont formées par un procédé de formation de sphères d'au moins une partie de ladite composition.

8. Composition de combinaison selon l'une quelconque des revendications précédentes, dans laquelle ledit agent assurant une libération contrôlée est au moins un ou un mélange d'éthyl cellulose, de polyméthacrylate, de poly(oxyde d'éthylène), de béhénate de glycéryle, d'hydroxypropyl méthyl cellulose, d'hydroxypropyl cellulose, de gomme de xanthane, de stéaroyl polyoxyl glycérides et d'alginate de sodium.

9. Composition de combinaison selon l'une quelconque des revendications précédentes, dans laquelle ladite charge est au moins un ou un mélange d'amidon, de mannitol, de phosphate de calcium dibasique, de lactose, d'amidon, de dihydrate d'hydrogéno phosphate de calcium, d'anhydrate d'hydrogéno phosphate dicalcique, de trihydrate de phosphate de calcium, de dioxyde de silicium et de glucose, le mannitol et/ou le phosphate de calcium dibasique étant préférés.

10. Composition de combinaison selon l'une quelconque des revendications précédentes, dans laquelle la dimension de particule desdites pastilles se situe dans la plage de 400 à 2000 µm, de préférence de 400 à 1500 µm, de façon davantage préférée de 450 à 1000 µm.

11. Composition de combinaison selon l'une quelconque des revendications précédentes, dans laquelle ledit liant est la polyvinylpyrrolidone.

12. Composition de combinaison selon l'une quelconque des revendications précédentes, dans laquelle ledit désintégrant est la croscarmellose sodique.

13. Composition de combinaison selon l'une quelconque des revendications précédentes, dans laquelle ledit agent de glissement est au moins un ou un mélange de dioxyde de silicium colloïdal, de talc, de silicate d'aluminium et de silicate de magnésium, le dioxyde de silicium colloïdal étant préféré.

14. Composition de combinaison selon l'une quelconque des revendications précédentes, dans laquelle ledit lubrifiant est le stéarate de magnésium.

15. Composition de combinaison selon l'une quelconque des revendications précédentes, consistant en
a. couche à libération immédiate
i. vildagliptine à raison de 3,5 à 60 % en poids ;
ii. mannitol à raison de 5 à 90 % en poids ;
iii. croscarmellose sodique à raison de 6 à 25 % en poids ;
iv. polyvinylpyrrolidone à raison de 0,1 à 25 % en poids ;
v. dioxyde de silicium à raison de 0,1 à 0,2 % en poids ;
vi. stéarate de magnésium à raison de 0,25 à 2 % en poids ;
b. couche à libération contrôlée
i. gliclazide à raison de 15 à 60 % en poids ;
ii. polyvinylpyrrolidone à raison de 0,1 à 25 % en poids ;
iii. phosphate de calcium dibasique à raison de 5 à 90 % en poids ;
iv. éthyl cellulose ou polyméthacrylate à raison de 0,5 à 40 % en poids ;
v. citrate de triéthyle à raison de 1 à 40 % en poids ;
vi. dioxyde de silicium à raison de 0,1 à 0,2 % en poids ;
vii. stéarate de magnésium à raison de 0,25 à 2 % en poids ;
c. couche d'enrobage
i. capsule d'hydroxypropyl méthyl cellulose à raison de 1 à 5 % en poids.

16. Composition de combinaison selon l'une quelconque des revendications précédentes, consistant en
a. couche à libération immédiate
i. vildagliptine à raison de 3,5 à 60 % en poids ;
ii. mannitol à raison de 5 à 90 % en poids ;
iii. polyvinylpyrrolidone à raison de 0,1 à 25 % en poids ;
iv. dioxyde de silicium à raison de 0,1 à 0,2 % en poids ;
v. stéarate de magnésium à raison de 0,25 à 2 % en poids ;
b. couche à libération contrôlée
i. gliclazide à raison de 15 à 60 % en poids ;
ii. polyvinylpyrrolidone à raison de 0,1 à 25 % en poids ;
iii. pastille de sucre à raison de 5 à 90 % en poids ;
iv. polyméthacrylate ou dérivés de celui-ci à raison de 0,5 à 40 % en poids (indépendant du pH) ;
v. phthalate de diéthyle à raison de 1 à 40 % en poids ;
vi. dioxyde de silicium à raison de 0,1 à 0,2 % en poids ;
vii. stéarate de magnésium à raison de 0,25 à 2 % en poids ;
c. couche d'enrobage
i. capsule d'hydroxypropyl méthyl cellulose à raison de 1 à 5 % en poids.

17. Procédé de préparation d'une formulation pharmaceutique selon l'une quelconque des revendications précédentes, comprenant les étapes consistant à :
a. tamiser de la vildagliptine, de la croscarmellose sodique et du mannitol, et mélanger dans un granulateur à cisaillement élevé ;
b. granuler le mélange résultant avec une solution de polyvinylpyrrolidone alcoolique/hydroalcoolique ;
c. tamiser les granules humides formées, les sécher et tamiser les granules séchées à nouveau ;
d. tamiser du glicazide et du phosphate de calcium dibasique et mélanger dans un granulateur à cisaillement élevé ;
e. granuler le mélange résultant avec une solution d'éthyl cellulose/polyvinylpyrrolidone hydroalcoolique ;
f. extruder les granules formées en sphères pour donner des pastilles ;
g. enrober les pastilles obtenues ci-dessus par une solution d'éthyl cellulose/polyméthacrylate alcoolique/hydroalcoolique contenant du citrate de triéthyle ;
h. ajouter d'abord du dioxyde de silicium puis du stéarate de magnésium aux granules contenant de la vildagliptine et aux granules enrobées contenant du gliclazide et mélanger le mélange résultant ;
i. remplir des capsules par les granules et pastilles obtenues ci-dessus.

18. Procédé de préparation d'une formulation pharmaceutique selon l'une quelconque des revendications précédentes, comprenant les étapes consistant à :
a. tamiser de la vildagliptine, de la croscarmellose sodique et du mannitol, et mélanger dans un granulateur à cisaillement élevé ;
b. granuler le mélange résultant avec une solution de polyvinylpyrrolidone alcoolique/hydroalcoolique ;
c. tamiser les granules humides formées, les sécher et tamiser les granules séchées à nouveau ;
d. préparer une solution de polyvinylpyrrolidone hydroalcoolique de gliclazide et enrober les pastilles de sucre avec cette solution ;
e. préparer une solution alcoolique/hydroalcoolique d'éthyl cellulose/polyméthacrylate et de phtalate de diéthyle, et enrober les pastilles de sucre contenant du gliclazide ;
f. ajouter d'abord du dioxyde de silicium puis du stéarate de magnésium aux granules contenant de la vildagliptine et aux pastilles enrobées contenant du glilcazide et mélanger le mélange résultant ;
g. remplir des capsules par les granules et pastilles obtenues ci-dessus.

19. Compositon de combinaison selon l'une quelconque des revendications précédentes pour prévenir ou traiter le diabète sucré chez les mammifères et en particulier chez les êtres humains.
